**Europäisches Patentamt**

**European Patent Office**

**Office européen des brevets**

(19)

(11) Veröffentlichungsnummer : **0 358 951 B1**

(12)
# EUROPÄISCHE PATENTSCHRIFT

(45) Veröffentlichungstag der Patentschrift :
**13.05.92 Patentblatt 92/20**

(51) Int. Cl.⁵ : **A61K 31/52,** A61K 9/14,
A61K 47/24, A61K 47/26

(21) Anmeldenummer : **89114814.0**

(22) Anmeldetag : **10.08.89**

(54) **Pulvrige, hydrophile Theophyllinformulierung und Verfahren zu ihrer Herstellung.**

(30) Priorität : **12.08.88 DE 3827362**

(43) Veröffentlichungstag der Anmeldung :
**21.03.90 Patentblatt 90/12**

(45) Bekanntmachung des Hinweises auf die
Patenterteilung :
**13.05.92 Patentblatt 92/20**

(84) Benannte Vertragsstaaten :
**AT BE CH DE FR GB IT LI NL SE**

(56) Entgegenhaltungen :
**DE-A- 1 492 203**
**GB-A- 932 874**
**CHEMICAL ABSTRACTS, Band 109, Nr. 22, 28.**
**November 1988, Seite 456, Zusammenfassung**
**Nr. 197189s, Columbus, Ohio, US; &JP-A-62**
**286 921 (TAISHO PHARMACEUTICAL CO. LTD)**
**12-12-1987**

(56) Entgegenhaltungen :
**CHEMICAL & PHARMACEUTICAL BULLETIN,**
**Band 35, Nr. 10, Oktober 1987, Seiten**
**4271-4276, Pharmaceutical Society of Japan,-**
**Tokyo, JP; T. NISHIHATA et al.: "Use of Hydro-**
**genated soya phospholipids as a diluent:**
**Preparation of sustained-release tablets oft-**
**heophylline and sodium diclofenac"**

(73) Patentinhaber : **BASF Aktiengesellschaft**
**Carl-Bosch-Strasse 38**
**W-6700 Ludwigshafen (DE)**

(72) Erfinder : **Horn, Dieter, Dr.**
**Schroederstrasse 69**
**W-6900 Heidelberg (DE)**
Erfinder : **Krueger, Goetz, Dr.**
**Wilhelmstrasse 54**
**W-5100 Aachen (DE)**
Erfinder : **Spengler, Reinhard, Dr.**
**Comenius Strasse 6**
**W-6700 Ludwigshafen (DE)**

EP 0 358 951 B1

## Beschreibung

Die Erfindung betrifft ein Verfahren zur Herstellung einer festen oralen Theophyllinformulierung, die sich, vor der Applikation in Wasser dispergiert, durch eine schnelle Wirkstoffanflutkinetik auszeichnet und den bitteren Theophyllingeschmack weitgehend vermeidet.

Theophyllin sowie seine wasserlöslichen Salze mit organischen Basen sind starke Bronchospasmolytika und finden wegen ihrer gefäßerweiternden Wirkung auch breite Verwendung in der Herz-Kreislauf-Therapie.

Ein lange bekanntes Problem bei der oralen Verabreichung von reinem Theophyllin sind schwankende Resorption und Bioverfügbarkeit aufgrund der geringen Wasserlöslichkeit des Wirkstoffs (ca. 0,5 % in sauren und neutralen pH-Bereichen). Diese Tatsache wiegt um so schwerer, wenn man die geringe therapeutische Breite der Substanz berücksichtigt. Es sind aus diesem Grund eine Reihe von Anstrengungen unternommen worden, diese Probleme durch Einsatz spezieller Theophyllinderivate bzw. durch geeignete Formulierungen zu beseitigen. Es wurden dabei verschiedene Wege beschritten:

Die Salze des Theophyllins mit starken organischen Basen (Ethylendiamin, Piperazin, Arginin- und Lysin-Derivate, Cholin u.a.) sind meist sehr gut wasserlöslich, jedoch sind solche wäßrigen Lösungen nur als Injektionslösungen sinnvoll zu verwenden; bei oraler Aufnahme kommt es im sauren Magenmilieu zur Freisetzung und Ausfällung des Wirkstoffs mit den bekannten Problemen der Bioverfügbarkeit. Außerdem können diese stark alkalischen Derivate des Theophyllins zu Schleimhautirritationen führen.

Ein anderer Lösungsansatz ist die Kombination von Theophyllin mit organischen Komplexbildnern oder Lösungsvermittlern (z.B. 7-Oxypropyl-Xanthin-Derivate, o-Carbamoylphenoxyessigsäure, Benzylalkohol usw.; vgl. z.B. DE-OS 26 34 335; Helwig, Moderne Arzneimittel, Wissenschaftliche Verlagsgesellschaft Stuttgart, 5. Aufl., 1980; D.S. Sitar, J.H. Nadeau, J.R. Ruedy, Cur. Ther. Res. 21, 233, 1977). Viele dieser Präparate haben jedoch den Nachteil, daß die verwendeten Hilfsstoffe auch als Wirkstoffe angesehen werden müssen, so daß es sich im strengen Sinne um Kombinationspräparate handelt, bei denen der eingentliche Wirkstoff (Theophyllin) häufig auch mengenmäßig nur eine untergeordnete Rolle spielt.

Weiterhin sind Präparate im Handel mit wäßrig/alkoholischen Theophyllin-Lösungen, die jedoch aufgrund ihres Alkohol-Gehaltes bedenklich und für die Behandlung von Kleinkindern und Säuglingen ungeeignet sind.

Als weitere Möglichkeit bietet sich die chemische Modifizierung des Theophyllins an. Eine Substitution am Theophyllin-Molekül in 7-Stellung führt ggf. zu sehr gut wasserlöslichen und fast neutral reagierenden Derivaten (z.B. Hydroxyethyltheophyllin, Hydroxypropyltheophyllin, Dihydroxypropyltheophyllin). Allerdings sind diese Derivate schwächer wirksam als Theophyllin, auch ist die zentralerregende Wirkung geringer (Helwig, loc. cit.).

Der Erfindung lag daher die Aufgabe zugrunde, eine Theophyllinformulierung zu entwickeln, die diese Nachteile überwindet.

Die Lösung dieser Aufgabe besteht in einer pulvrigen, hydrophilen Theophyllinformulierung, bestehend aus
a) 5 bis 15, vorzugsweise 8 bis 12 Gew.-% Theophyllin,
b) 15 bis 30, vorzugsweise 20 bis 25 Gew.-% Lecithin,
c) 45 bis 80, vorzugsweise 55 bis 70 Gew.-% eines Zuckers,
d) 0 bis 5, vorzugsweise 1 bis 3 Gew.-% eines Fließmittels und
e) 0 bis 30, vorzugsweise 0,1 bis 5 Gew.-% weiterer Zusätze sowie in einem Verfahren zu deren Herstellung, wobei bei 70 bis 100, vorzugsweise 80 bis 85°C der Zucker in einer wäßrigen Theophyllinlösung gelöst wird, dann das Lecithin und ggf. ein Farbstoff, Geschmacksstoff, Stabilisierungsmittel und/oder weitere Zusätze in der Lösung dispergiert und ggf. gelöst werden, die heiße Lösung sprühgetrocknet, das trockene Pulver gesiebt und ggf. mit einem Fließmittel vermischt und die fertige Mischung ggf. agglomeriert wird.

Als Zucker wird Saccharose bevorzugt, doch kommt im Prinzip jeglicher Zucker in Betracht.

Als Fließmittel oder Fließverbesserer wird poröses Silikagel bevorzugt, andere sind aber ebenfalls brauchbar, beispielsweise Stärke, Maltodextrin, Polyvinylpyrrolidon.

Als weitere Zusatzstoffe kommen z.B. Lebensmittelfarbstoffe, Geschmacksstoffe, Stabilisatoren gegen Oxidation, beispielsweise $\alpha$-Tocopherol oder Ascorbylpalmitat, ferner Streckmittel und als solche vor allem wiederum Zucker, in Betracht.

Gemäß Takashima et al., C.A. 109 (1988), 197 189s, werden Theophyllin-Retard-Präparate hergestellt durch Überziehen großer Theophyllin-Kristalle mit Lecithin-Pulver, einem Bindemittel und einer pulvrigen hydrophoben Substanz und Erwärmen.

GB-932 871 beschreibt die Herstellung von theophyllinhaltigen Fertigsäften Dabei wird Guajacol und ggf. Zucker zur Löslichkeitserhöhung eingesetzt.

Bei einer besonders bevorzugten Ausführungsform der Erfindung handelt es sich um eine Sprüheinbettung des Theophyllins in eine hydrophile Matrix, die im wesentlichen aus Zucker und raffiniertem (gereinigtem) Sojalecithin besteht. Das fertige Produkt ist ein Trockenpulver mit einem Theophyllingehalt von ca. 10 %.

Aus diesem Trockenpulver lassen sich durch Einrühren in Wasser oder andere wäßrige Flüssigkeiten wie Milch, Säfte, Tee usw. leicht Dispersionen mit Wirkstoffgehalten bis zu 20 g/l herstellen. Diese Dispersionen zeigen, obwohl sie Konzentrationen weit über der Sättigungsgrenze enthalten, überraschenderweise keine Wirkstoffabscheidungen über ausreichend lange Zeiträume (mehr als 30 min).

Wie Probandenversuche mit subtherapeutischen Dosen ergeben haben, zeigt das Theophyllin in den erfindungsgemäßen Zubereitungen Plasmaanflutkinetiken, die mit denen der in der Literatur beschriebenen schnell freisetzenden Formen vergleichbar sind, jedoch ohne deren eingangs geschilderten Nachteile. Besonders überraschend ist, daß der notorische penetrant bittere Geschmack des Theophyllins, der allen bisher bekannten Präparaten anhaftet, bei der erfindungsgemäßen Formulierung selbst ohne Geschmacksverbesserer weitgehend verschwunden ist. Dies ist ein ganz wesentlicher Vorteil. Durch Zumischen geringer Mengen eines Aromastoffes können verschiedene Geschmacksrichtungen eingestellt werden.

Außerdem ist die Mischung alkoholfrei, daher auch für Kleinkinder geeignet.

Die Kombination dieser Merkmale ergibt ein Eigenschaftsprofil, das in dieser Form bisher nicht realisiert werden konnte.

Die Wirkstoffaufname wurde auf folgende Weise untersucht:

7 gesunden Probanden wurden jeweils 1073 mg Trockenpulver, entsprechend 100 mg Theophyllin, oral verabreicht. Die sich ergebenden Plasmaspiegel wurden analysiert; eine Kinetik wurde ermittelt.

Um zu verhindern, daß das Pulver beim Einrühren in wäßrige Flüssigkeiten aufschwimmt, kann es zweckmäßig sein, es zu agglomerieren (verdichten). Dies kann z.B. dadurch geschehen, daß man das Pulver zwischen zwei Walzen durchlaufen läßt. Man erhält dann eine dünne Schülpe, also ein sehr leicht zerbröselndes Preßfell, das beim Sieben ein agglomeriertes (verdichtetes) Pulver ergibt. Eine andere Möglichkeit zum Agglomerieren besteht z.B. im Aufdüsen von sehr wenig Wasser auf das bewegte Pulver (z.B. Wirbelbett).

Beispiel 1

Zu einer Lösung von 90 g Theophyllin in 3000 g Wasser von 80 bis 85°C wurden 405 g Saccharose unter Rühren zugegeben. Nach erfolgter Auflösung wurden 405 g ®Megglezitin (Soja-Lecithinzubereitung der Firma Meggle, Wasserburg am Inn) portionsweise eingetragen und mit einem leistungsfähigen Homogenisator (Ultraturrax) dispergiert. Ein Gew.-%, bezogen auf Lecithin, α-Tocopherol wurde ebenfalls an dieser Stelle einhomogenisiert. Während des gesamten Prozesses wurde die Temperatur bei 80 bis 85°C gehalten. Die heiße Suspension wurde anschließend einer Sprühtrocknung unterzogen, das Trockenpulver wurde durch ein Sieb mit der Maschenweite 710 μm gesiebt und und zur Verbesserung der Fließeigenschaften mit 18 g ®Aerosil 200 der Firma Degussa versetzt.

Das fertige Produkt war ein helles, freifließendes Pulver folgender Zusammensetzung:
Theophyllin: 9,8 %
®Megglezitin: 44,1 %
Saccharose: 44,1 %
Aerosil 200: 2 %.

**Patentansprüche**

1. Pulvrige, hydrophile Theophyllinformulierung, bestehend aus
a) 5 bis 15 Gew.-% Theophyllin,
b) 15 bis 30 Gew.-% Lecithin,
c) 45 bis 80 Gew.-% eines Zuckers,
d) 0 bis 5 Gew.-% eines Fließmittels und
e) 0 bis 30 Gew.-% weiterer Zusätze.

2. Verfahren zur Herstellung einer pulvrigen, hydrophilen Theophyllinformulierung nach Anspruch 1, dadurch gekennzeichnet, daß
a) bei 70 bis 100°C der Zucker in einer wäßrigen Theophyllinlösung gelöst wird, dann das Lecithin und ggf. ein Farbstoff, Geschmackstoff, Stabilierungsmittel und/oder weitere Zusätze in der Lösung dispergiert und ggf. gelöst werden,
b) die heiße Lösung sprühgetrocknet,
c) das trockene Pulver gesiebt und ggf.
d) mit einem Fließmittel vermischt und
e) die fertige Mischung ggf. agglomeriert wird.

**Claims**

1. A hydrophilic theophylline powder formulation consisting of
a) from 5 to 15% by weight of theophylline,
b) from 15 to 30% by weight of lecithin,
c) from 45 to 80% by weight of a sugar,
d) from 0 to 5% by weight of a flow agent and
e) from 0 to 30% by weight of further additives.

2. A process for the preparation of a hydrophilic theophylline powder formulation as claimed in claim 1, wherein
a) the sugar is dissolved in an aqueous theophylline solution at from 70 to 100°C, the lecithin and, if required, a colorant, a flavoring, a stabilizer and/or further additives are then dispersed and, where relevant, dissolved in the solution,
b) the hot solution is spray-dried,
c) the dry powder is screened and, if required,
d) mixed with a flow agent and
e) if required, the ready-prepared mixture is agglomerated.

**Revendications**

1. Composition pulvérulente hydrophile de théophylline consistant en :
a) 5 à 15 % en poids de théophylline,
b) 15 à 30 % en poids de lécithine,
c) 45 à 80 % en poids d'un sucre,
d) 0 à 5 % en poids d'un agent d'écoulement, et
e) 0 à 30 % en poids d'autres additifs.

2. Procédé de préparation d'une composition pulvérulente hydrophile de théophyline selon la revendication 1, caractérisé en ce que :
a) on dissout le sucre à une température de 70 à 100 degrés C dans une solution aqueuse de théophylline, on disperse ensuite et le cas échéant on dissout dans la solution la lécithine et éventuellement un colorant, un arôme, un stabilisant et/ou d'autres additifs,
b) on sèche la solution chaude par atomatisation,
c) on tamise la poudre sèche et,le cas échéant,
d) on la mélange avec un agent d'écoulement, et
e) on agglomère éventuellement le mélange fini.